# FASCICULE DE BREVET EUROPEEN

(11) **EP 2 177 449 B1**
(45) Date de publication et mention de la délivrance du brevet: **25.02.2015**
(21) Numéro de dépôt: 09173016.8
(22) Date de dépôt: 14.10.2009
(51) Int. Cl.: B65D 47/18, B65D 47/20, A61J 1/14, B05B 11/04, B05B 11/00

(54) **Dispositif de distribution de liquide muni d'un organe d'étanchéité ayant une partie élastomère**
Vorrichtung zur Verteilung von Flüssigkeit, die mit einem Dichtungsorgan mit einem Elastomerteil ausgestattet ist
Liquid distribution device equipped with a sealing element with an elastomer part

(30) Priorité: 15.10.2008 FR 0805716
(43) Date de publication de la demande: 21.04.2010
(73) Titulaire: NEMERA LA VERPILLIERE, 38290 La Verpillière (FR)
(72) Inventeur: Painchaud, Gaëtan, 69340, FRANCHEVILLE (FR); Grevin, Guillaume, 38080, L'ISLE D'ABEAU (FR); Julia, Xavier, 38090, VILLEFONTAINE (FR); Lanzi, Sylvain, 38850, CHIRENS (FR)
(74) Mandataire: Thiollier, Clémence-Olivia Laure Marie

(56) Documents cités:
- EP-A- 1 052 023
- WO-A1-2007/056233
- US-A- 5 183 184

## Description

La présente invention concerne le domaine technique de la distribution de liquide, sous forme de gouttes, par exemple de liquide ophtalmique.

On connaît déjà dans l'état de la technique des dispositifs permettant de mettre en oeuvre cette distribution. Selon un exemple décrit dans le document WO2007/056233, qui décrit un dispositif selon le préambule de la revendication 1, le dispositif peut comprendre une valve assurant une étanchéité, réalisée dans un matériau élastomère et pouvant prendre dans le dispositif une position de libération du liquide et une position de blocage du liquide. Plus précisément, cette valve prend sa position de libération de liquide sous l'effet d'une pression exercée par l'utilisateur sur le flacon du dispositif.

La présente invention vise notamment à proposer un dispositif de distribution dans lequel l'organe d'étanchéité, dans sa position de blocage, assure le blocage du liquide de façon plus fiable.

A cet effet, l'invention a pour objet un dispositif de distribution de liquide, sous forme de gouttes, comprenant un organe d'étanchéité pouvant prendre une position de libération du liquide et une position de blocage du liquide, dans lequel l'organe d'étanchéité comprend une partie élastomère et une partie rigide, ces parties étant solidaires l'une de l'autre en déplacement, la partie élastomère assurant l'étanchéité de l'organe d'étanchéité.

On entend par "partie élastomère" une partie comprenant ou réalisée dans un matériau élastomère. La partie élastomère et la partie rigide sont réalisées dans un matériau différent, la partie élastomère étant plus élastique que la partie rigide. Par ailleurs, on comprend que l'organe d'étanchéité défini ci-dessus assure une étanchéité du dispositif. On pourrait également le désigner "valve d'étanchéité".

Ainsi, les inventeurs ont eu l'idée de prévoir un organe d'étanchéité qui est réalisé, non pas intégralement dans un matériau élastomère, mais qui comprend une partie élastomère couplée à une partie rigide. Il en résulte que l'organe d'étanchéité a une tenue plus constante dans le temps, ce qui procure une meilleure étanchéité du dispositif. En effet, dans le cas d'une valve réalisée intégralement en élastomère, on constate un certain fluage ou relaxation du matériau élastomère dans le temps, ce qui dégrade l'étanchéité de la valve.

En outre, ce fluage ou cette relaxation sont d'autant plus importants lorsqu'on exerce une contrainte mécanique sur la valve pour lui faire prendre sa position blocage ou de libération de liquide. Aussi, grâce à la partie rigide sur l'organe d'étanchéité, on peut exercer la contrainte sur la partie rigide, cette contrainte étant transmise à la partie élastomère, tout en évitant le fluage ou toute autre déformation de la matière élastomère dus au contact entre la source de la contrainte et l'élastomère. De plus, la présence de la partie rigide autorise à exercer une contrainte plus importante sur l'organe d'étanchéité. En effet, dans le cas où l'organe d'étanchéité est intégralement réalisé en matériau élastomère, il est délicat d'exercer un appui de grande intensité sur l'organe d'étanchéité, du fait que cet appui, non seulement risque de dégrader l'organe d'étanchéité, mais est en outre amorti par le matériau élastomère, et perd donc en intensité.

On notera par ailleurs que l'organe d'étanchéité proposé assure plus facilement l'étanchéité que dans le cas où il est intégralement réalisé dans un matériau relativement rigide sur lequel on appui. En effet, dans ce cas, il est nécessaire d'exercer une contrainte très importante sur l'organe d'étanchéité pour qu'il assure l'étanchéité. Ici, grâce à la partie élastomère qui assure l'étanchéité, la contrainte exercée peut être moins importante.

Ainsi, grâce à l'utilisation conjointe d'un matériau rigide et d'un matériau élastomère, on bénéficie des avantages de chacun des matériaux, tout en diminuant les inconvénients de chacun.

Le dispositif peut en outre comporter l'une ou plusieurs des caractéristiques suivantes.
- La partie élastomère comprend un matériau élastomère, tel qu'un thermoplastique élastomère, par exemple le matériau commercialisé sous la marque "Santoprene", ou encore tel qu'un silicone, matériau apprécié pour ses qualités de faible déformation dans le temps.
- La partie rigide est de préférence réalisée en matière thermoplastique non susceptible de se déformer sous l'effet d'une contrainte telle que celles exercées dans le dispositif de distribution du liquide, par exemple en polypropylène (PP), en polyéthylène haute densité (PEHD), ou encore en polybutylène téréphtalate (PBT).
- La partie élastomère et la partie souple sont assemblées par surmoulage, par soudage, par collage, par encliquetage ou encore par rivetage. Néanmoins, tout autre moyen d'assemblage permettant de rendre ces parties solidaires l'une de l'autre en déplacement est envisageable.
- Le dispositif comporte un élément de rappel, exerçant une force de rappel sur l'organe d'étanchéité pour qu'il prenne sa position de blocage de liquide, l'élément de rappel étant de préférence en appui contre ou fixé à la partie rigide de l'organe d'étanchéité. Ainsi, la partie rigide permet de recevoir la contrainte de l'élément de rappel sur l'organe d'étanchéité, et évite ainsi que cette contrainte soit exercée directement sur la partie élastomère, en risquant de la déformer. L'appui sur la partie rigide est de préférence direct.
- L'élément de rappel est choisi parmi un ressort, éventuellement un ressort en spirale, une lame élastique, ou encore un élément réalisé en matériau élastomère. Cet élément de rappel peut aussi bien être réalisé dans un matériau métallique que dans un matériau non métallique.
- L'élément de rappel est venu de matière avec la partie élastomère ou la partie rigide de l'organe d'étanchéité, ou encore avec une autre partie fonctionnelle de l'embout, par exemple avec une enveloppe de l'embout. Ainsi, l'élément de rappel peut être directement intégré dans l'une de ces parties, ce qui a pour avantage de diminuer le nombre de pièces du dispositif.
- L'organe d'étanchéité, de préférence sa partie rigide, comprend des moyens de formation de goutte de liquide. Ainsi, on utilise l'organe d'étanchéité pour réaliser les gouttes de liquide, ce qui a pour avantage de ne pas prévoir de tels moyens sur une autre pièce séparée de l'organe d'étanchéité, et donc d'avoir à prévoir une étanchéité entre l'organe d'étanchéité et les moyens de formation de la goutte. On notera que les moyens de formation de la goutte peuvent prendre la forme d'une partie évasée sur laquelle débouche un canal de distribution de liquide réalisé dans la partie élastomère, cette forme évasée, par exemple un cône, évitant que la goutte soit délivrée sous la forme de jet.
- La partie rigide recouvre la partie élastomère sur une grande partie de la surface de la partie élastomère. Ainsi, la partie rigide constitue une sorte de doublure de la partie élastomère, ce qui a pour effet notamment de répartir une contrainte, exercée ponctuellement, sur l'ensemble de la surface de la partie rigide, donc de la partie élastomère. II en résulte que la partie élastomère, peu soumise à des contraintes locales, est moins susceptible de se déformer.
- La partie élastomère comporte une zone non recouverte par la partie rigide, ce qui peut libérer cette zone pour qu'elle puisse s'allonger élastiquement, par exemple pour permettre à l'organe d'étanchéité de prendre sa position de libération de liquide.
- La partie élastomère et la partie rigide ont chacune la forme générale d'un chapeau muni d'une forme centrale cylindrique prolongée par un rebord de rattachement de l'organe à l'embout.
- La partie élastomère, dans sa position de blocage de liquide, coopère avec des moyens d'appui, ces moyens d'appui étant réalisés sur un noyau intérieur du dispositif, éventuellement sous forme d'une saillie. C'est la coopération entre la partie élastomère et ces moyens d'appui qui bloque le liquide.

L'invention sera mieux comprise à la lecture de la description qui va suivre, donnée uniquement à titre d'exemple et faite en se référant aux dessins dans lesquels :
- la figure 1 est une vue schématique illustrant fonctionnellement un dispositif selon un mode de réalisation de l'invention ;
- la figure 2 est une vue en coupe longitudinale illustrant un exemple de dispositif tel que schématisé sur la figure 1.

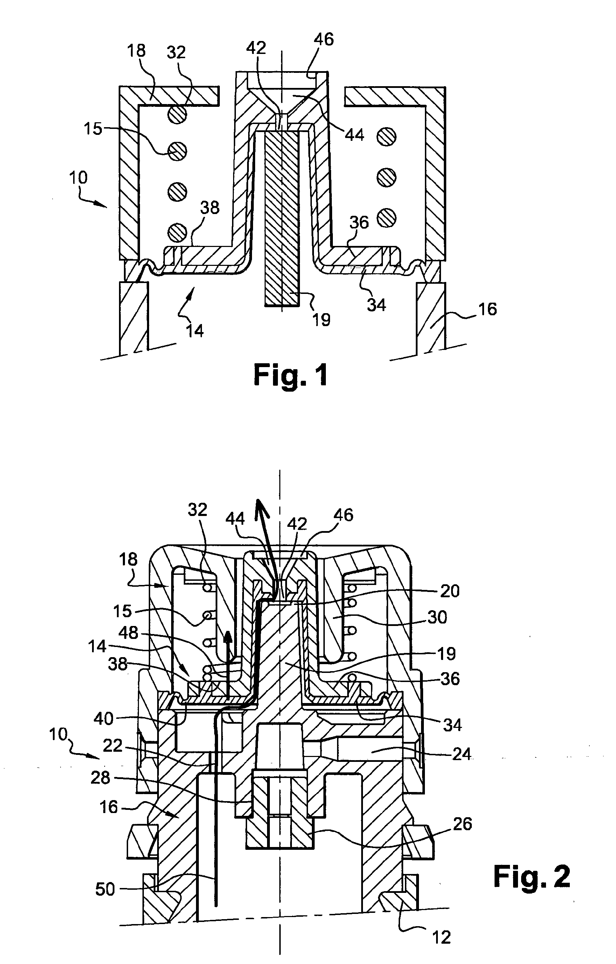

Un dispositif de distribution de liquide comprend un embout 10, représenté partiellement et schématiquement sur la figure 1, destiné à être rapporté sur un réservoir en matière plastique contenant le liquide à distribuer. Le dispositif permet de distribuer des doses prédéterminées de liquide, plus précisément des gouttes de liquide, destinées à une application oculaire, nasale ou buccale, par exemple des gouttes de collyre pour les yeux.

Comme on peut le voir sur la figure 2, l'embout 10 est rapporté sur le col 12 du réservoir, ce réservoir étant destiné à être pressé par l'utilisateur pour faire sortir le liquide, comme cela est décrit dans la suite. Par ailleurs, l'embout 10 est recouvert d'un capuchon de fermeture, non représenté sur les figures. Dans cet exemple, le réservoir est en matière plastique et est destiné à être pressé par l'utilisateur pour faire sortir le liquide. On peut envisager d'autres types de réservoir, notamment en verre ou en métal, l'utilisateur pouvant libérer du liquide par une autre action qu'en le pressant, par exemple en appuyant sur un élément d'activation d'une pompe.

L'embout 10 comprend un organe d'étanchéité 14, disposé entre une première partie 16 et une deuxième partie 18 de l'embout 10, ainsi qu'un élément de rappel 15.

La première partie 16 est un noyau intérieur 16, comprenant une protubérance 19 de forme sensiblement cylindrique et faisant saillie de l'extrémité distale du noyau 16. Sur son extrémité distale, cette protubérance 19 porte des moyens 20 d'appui sur l'organe d'étanchéité 14, ces moyens 20 étant dans cet exemple composés d'une saillie formant un bourrelet annulaire destinée à appuyer sur l'organe d'étanchéité 14. De manière alternative, les moyens 20 peuvent être directement constitués par l'extrémité distale de la protubérance 19, ou bien prendre d'autres formes. Le noyau intérieur 16 comporte par ailleurs un canal 22 de passage du liquide depuis le réservoir vers l'extérieur du dispositif, ainsi qu'une partie de connexion du noyau 16 au réservoir 12. L'embout 10 comporte par ailleurs des moyens d'évent, destinés à faire passer de l'air à l'intérieur du réservoir pour combler une dépression générée par l'appui de l'utilisateur pour faire sortir du liquide. Dans cet exemple, les moyens d'évent sont portés par le noyau intérieur 16 et comprennent un canal 24 de passage de l'air, traversé par un filtre hydrophobe 26, destiné à filtrer l'air entrant sans pour autant permettre à du liquide de s'échapper par le canal 24. Plus précisément dans cet exemple, le filtre 26 est disposé dans un logement 28 de réception du filtre, ce logement 28 prenant la forme d'une rainure annulaire disposée au centre de la surface intérieure du noyau 16, dans laquelle le filtre 26 est encastré.

La deuxième partie 18 de l'embout 10 correspond, dans cet exemple, à une enveloppe supérieure extérieure de l'embout 10. Cette enveloppe extérieure 18 est destinée à coiffer le noyau intérieur 16, l'organe d'étanchéité 14 (au moins partiellement) et le ressort 15. Plus précisément, elle comprend une protubérance intérieure ouverte 30, formée par une rainure annulaire centrale, destinée à entourer l'extrémité distale de la protubérance 18 et de l'organe d'étanchéité 14 de façon à permettre le passage de liquide hors du dispositif. L'enveloppe 18 comporte en outre un siège 32 d'appui de l'élément de rappel 15, ce siège 32 étant disposé autour de la protubérance 30.

L'organe d'étanchéité 14 peut prendre une position de blocage du liquide, illustrée sur les figures 1 et 2, empêchant le retour du liquide une fois sorti de la zone étanche, et une position de libération du liquide (non représentée). L'organe 14 comporte une partie élastomère 34 et une partie rigide 36, les parties 34 et 36 étant solidaires l'une de l'autre en déplacement, c'est-à-dire que lorsque la partie 34 se déplace, la partie 36 se déplace également avec elle, et vice-versa. Dans cet exemple, les parties 34 et 36 sont assemblées par surmoulage, mais l'on pourrait envisager d'autres types d'assemblage. La partie élastomère 34 est réalisée dans un matériau élastomère, tel que du silicone ou un matériau thermoplastique élastomère. La partie rigide 36 est réalisée dans un matériau thermoplastique tel que du polypropylène. La partie rigide 36 comporte une surface 38 d'appui de l'élément de rappel 15. Comme on peut le constater sur les figures, la partie rigide 36 recouvre la partie élastomère 34 sur sensiblement toute sa surface, une zone 40 de la partie élastomère étant néanmoins laissée libre à l'extrémité de la partie élastomère, de façon à permettre l'allongement de cette partie élastomère 34. Plus précisément, la partie élastomère 34, ainsi que la partie rigide 36, ont chacune la forme d'un chapeau muni d'une forme centrale cylindrique, de forme sensiblement complémentaire à celle de la protubérance 19 du noyau 16, cette forme cylindrique étant prolongée sur son extrémité proximale par un rebord, l'un des rebords servant au rattachement de l'organe 14 à l'embout. Dans l'exemple décrit, le rebord de la partie élastomère 34 est rattaché, à sa périphérie 40, au reste de l'embout 10. Ainsi, la partie rigide 36 recouvre la partie élastomère 34 sur une grande partie de sa surface, sauf au niveau de sa périphérie 40. Comme on peut le voir sur les figures, les parties 34, 36 définissent un canal 42, ménagé sur le fond de leur forme centrale cylindrique, permettant au liquide de sortir. Par ailleurs, la partie rigide 36 comporte des moyens 44 de formation de gouttes de liquide. Plus précisément, ces moyens 44 ont la forme d'un cône partant du canal 42 et s'élargissant vers l'extrémité distale du dispositif, de façon à former une goutte et à éviter que le liquide soit distribué par jet, le cône 44 débouchant sur une portion cylindrique 46, permettant de calibrer la goutte.

L'élément de rappel 15 est, dans cet exemple, un ressort métallique en spirale. Cet élément 15 exerce une force de rappel sur l'organe d'étanchéité 14, en s'appuyant sur la surface 38 de la partie rigide 36, de façon à rappeler l'organe d'étanchéité 14 dans sa position de blocage du liquide.

Comme on peut le voir sur les figures, l'organe d'étanchéité 14 est fixé entre les deux parties 16, 18, de façon étanche (étanchéité statique), afin d'éviter que du liquide, passant par le canal 22, ne s'échappe à l'intérieur de l'enveloppe 18.

Le fonctionnement du dispositif de distribution va à présent être décrit.

Lorsque l'utilisateur souhaite distribuer des gouttes de liquide, il presse sur le réservoir du dispositif, ce qui a pour effet de faire passer du liquide dans le canal 22, et donc d'exercer une pression sur la partie élastomère 34. Sous cette pression, l'organe d'étanchéité passe de sa position de blocage de liquide à sa position de libération de liquide, en effectuant une translation vers le haut, comme illustré par la flèche 48. Plus précisément, la zone 40 de la partie élastomère 34 se déforme, en s'allongeant, pour autoriser ce déplacement vers le haut de la partie élastomère. A l'issue de ce déplacement, l'étanchéité assurée par la coopération des moyens d'appui 20 avec l'organe d'étanchéité 14 est rompue, et le liquide peut passer à travers le canal 42 jusque dans les parties 44, 46, de façon à former une goutte de liquide. Le trajet du liquide est illustré par la flèche 50. Une fois la goutte libérée, l'utilisateur peut arrêter d'exercer la pression sur le réservoir, lequel se remplit d'air grâce au passage au canal 24. Par ailleurs, la pression du liquide sortant s'arrêtant, l'organe d'étanchéité 14 reprend sa position de blocage du liquide, sous l'effet de la force de rappel de l'élément 15. Ainsi, les moyens d'appui 20 et la partie élastomère de l'organe 14 coopèrent à nouveau de façon à bloquer la sortie de liquide.

On notera que l'invention n'est pas limitée aux exemples précédemment décrits. En particulier, l'élément de rappel est, dans le mode de réalisation décrit, un ressort en spirale, mais l'on pourrait prévoir d'autres types d'éléments de rappel, en matière métallique ou pas, tels qu'une lame élastique ou un élément élastomère. Notamment, cet élément de rappel 15 pourrait être directement intégré dans l'enveloppe 18 de l'embout 10, ou dans l'organe d'étanchéité 14, en étant intégré soit dans la partie élastomère 34 soit dans la partie rigide 36. Ainsi en intégrant cet élément 15 dans une autre pièce fonctionnelle, on diminue le nombre de pièces à assembler.

Les avantages du dispositif de distribution ont été développés ci-dessus. On notera en particulier que la partie rigide 36 constitue une sorte de coquille pour la partie élastomère 34, ce qui permet d'exercer plus facilement une contrainte sur l'organe d'étanchéité, sans risquer de le déformer.

## Revendications

1. Dispositif de distribution de liquide sous forme de gouttes, comportant un organe d'étanchéité (14) pouvant prendre une position de libération du liquide et une position de blocage du liquide, dans lequel l'organe d'étanchéité (14) comprend une partie élastomère (34), **caractérisé en ce que** l'organe d'étanchéité (14) comprend de plus une partie rigide (36), la partie élastomère assurant l'étanchéité de l'organe d'étanchéité, ces parties (34, 36) étant solidaires l'une de l'autre en déplacement.

2. Dispositif selon la revendication 1, dans lequel les parties élastomère (34) et rigide (36) sont assemblées par surmoulage, par soudage, par collage, par encliquetage ou par rivetage.

3. Dispositif selon l'une quelconque des revendications précédentes, comportant un élément de rappel (15), exerçant une force de rappel sur la partie élastomère (34) pour qu'elle prenne sa position de blocage, l'élément de rappel (15) étant en appui contre ou fixé à la partie rigide (36) de l'organe d'étanchéité (14).

4. Dispositif selon la revendication précédente, dans lequel l'élément de rappel (15) est choisi parmi un ressort, éventuellement en spirale, une lame élastique, ou un élément réalisé en matériau élastomère.

5. Dispositif selon la revendication 3 ou 4, dans lequel l'élément de rappel est venu de matière avec la partie élastomère (34) ou avec la partie rigide (36) de l'organe d'étanchéité (14), ou encore avec une autre partie fonctionnelle de l'embout, par exemple avec une enveloppe (18) de l'embout.

6. Dispositif selon l'une quelconque des revendications précédentes, dans lequel la partie rigide (36) comprend des moyens (44, 46) de formation de gouttes de liquide.

7. Dispositif selon l'une quelconque des revendications précédentes, dans lequel la partie rigide (36) recouvre la partie élastomère (34) sur une grande partie de la surface de la partie élastomère (34).

8. Dispositif selon la revendication précédente, dans lequel la partie élastomère (34) comporte une zone (40) non recouverte par la partie rigide (36).

9. Dispositif selon l'une quelconque des revendications précédentes, dans lequel la partie élastomère (34) et la partie rigide (36) ont chacune la forme générale d'un chapeau muni d'une forme centrale cylindrique prolongée à une extrémité par un rebord, l'un des rebords (40) servant de rattachement de l'organe (14) à l'embout.

10. Dispositif selon l'une quelconque des revendications précédentes, dans lequel la partie élastomère (34), dans sa position de blocage de liquide, coopère avec des moyens (20) d'appui sur l'organe d'étanchéité (14), ces moyens d'appui étant réalisés sur un noyau intérieur (16) du dispositif, éventuellement sous forme d'une saillie (20).

## Patentansprüche

1. Vorrichtung zum Ausgeben von Flüssigkeit in Form von Tropfen, die ein Abdichtungsorgan (14) aufweist, das eine Position zum Freigeben der Flüssigkeit und eine Position zum Blockieren der Flüssigkeit einnehmen kann, wobei das Abdichtungsorgan (14) einen Elastomerteil (34) umfasst, **dadurch gekennzeichnet, dass** das Abdichtungsorgan (14) des Weiteren einen starren Teil (36) umfasst, wobei der Elastomerteil (34) die Abdichtung des Abdichtungsorgans sicherstellt, wobei diese Teile (34, 36) bewegungsfest miteinander verbunden sind.

2. Vorrichtung nach Anspruch 1, wobei der Elastomerteil (34) und der starre Teil (36) durch Aufformen, durch Verschweißen, durch Verkleben, durch Einrasten oder durch Vernieten zusammengefügt sind.

3. Vorrichtung nach einem der vorhergehenden Ansprüche, die ein Rückstellelement (15) aufweist, das eine Rückstellkraft auf den Elastomerteil (34) ausübt, damit er seine Blockierposition einnimmt, wobei sich das Rückstellelement (15) gegen den starren Teil (36) des Abdichtungsorgans (14) abstützt oder daran befestigt ist.

4. Vorrichtung nach dem vorhergehenden Anspruch, wobei das Rückstellelement (15) ausgewählt ist aus einer Feder, gegebenenfalls spiralförmig, einer Federlasche oder einem Element, das aus Elastomermaterial realisiert ist.

5. Vorrichtung nach Anspruch 3 oder 4, wobei das Rückstellelement einstückig mit dem Elastomerteil (34) oder mit dem starren Teil (36) des Abdichtungsorgans (14) oder auch mit einem anderen funktionalen Teil des Stutzens gebildet ist, zum Beispiel mit einer Hülle (18) des Stutzens.

6. Vorrichtung nach einem der vorhergehenden Ansprüche, wobei der starre Teil (36) Mittel (44, 46) zur Bildung von Flüssigkeitstropfen umfasst.

7. Vorrichtung nach einem der vorhergehenden Ansprüche, wobei der starre Teil (36) den Elastomerteil (34) über einen großen Teil der Oberfläche des Elastomerteils (34) abdeckt.

8. Vorrichtung nach dem vorhergehenden Anspruch, wobei der Elastomerteil (34) eine Zone (40) aufweist, die nicht durch den starren Teil (36) abgedeckt wird.

9. Vorrichtung nach einem der vorhergehenden Ansprüche, wobei der Elastomerteil (34) und der starre Teil (36) jeder die allgemeine Form einer Kappe haben, die mit einer zylindrischen mittigen Form versehen ist, die an einem Ende durch einen Bund verlängert ist, wobei einer der Bünde (40) als Befestigung des Organs (14) an dem Stutzen dient.

10. Vorrichtung nach einem der vorhergehenden Ansprüche, wobei der Elastomerteil (34), in seiner Position zum Blockieren der Flüssigkeit, mit Mitteln (20) zum Abstützen auf dem Abdichtungsorgan (14) zusammenwirkt, wobei diese Abstützmittel an einem inneren Kern (16) der Vorrichtung realisiert sind, gegebenenfalls in Form eines Vorsprungs (20).

## Claims

1. Device for dispensing liquid in drop form, comprising a sealing component (14) which can take up a liquid release position and a liquid blocking position, wherein the sealing component (14) comprises an elastomer part (34), **characterized in that** the sealing component (13) comprises a rigid part (36), the elastomer part providing the seal of the sealing component, these parts (34, 36) being fastened to each other, in particular when moving.

2. Device according to claim 1, wherein the elastomer (34) and rigid (36) parts are assembled by overmoulding, welding, gluing, clipping or riveting.

3. Device according to any of the preceding claims, comprising a return element (15), exerting a return force on the elastomer part (34) so that it takes up its blocking position, the return element (15) resting against or being fixed to the rigid part (36) of the sealing device (14).

4. Device according to the preceding claim, wherein the return element (15) is either a spring, possibly a helical spring, an elastic strip, or an element made from an elastomer material.

5. Device according to claim 3 or 4, wherein the return element is made in one piece with the elastomer part (34) or with the rigid part (36) of the sealing component (14), or with another functional part of the nozzle, for example with an envelope (18) of the nozzle.

6. Device according to any of the preceding claims, wherein the rigid part (36) comprises means (44, 46) for forming liquid drops.

7. Device according to any of the preceding claims, wherein the rigid part (36) covers the elastomer part (34) over a large proportion of the surface of the elastomer part (34).

8. Device according to the preceding claim, wherein the elastomer part (34) comprises a zone (40) not covered by the rigid part (36).

9. Device according to any of the preceding claims, wherein the elastomer part (34) and the rigid part (36) each have the overall shape of a hat with a central cylindrical shape, extended at one end by an edge, one of the edges (40) being used to attach the component (14) to the nozzle.

10. Device according to any of the preceding claims, wherein the elastomer part (34), in its liquid blocking position, cooperates with means (20) for pressing on the sealing component (14), these pressing means being formed on an inner core (16) of the device, possibly as a projection (20).
